# EUROPÄISCHE PATENTSCHRIFT

(11) **EP 1 594 449 B1**
(45) Veröffentlichungstag und Bekanntmachung des Hinweises auf die Patenterteilung: **13.08.2008**
(21) Anmeldenummer: 04710025.0
(22) Anmeldetag: 11.02.2004
(51) Int. Cl.: A61Q 5/00, A61K 8/89, A61K 8/67

(54) **HAARBEHANDLUNGSMITTEL**
HAIR TREATMENT AGENTS
AGENTS DE TRAITEMENT CAPILLAIRE

(30) Priorität: 19.02.2003 DE 10307116
(43) Veröffentlichungstag der Anmeldung: 16.11.2005
(73) Patentinhaber: Henkel AG & Co. KGaA, 40589 Düsseldorf (DE)
(72) Erfinder: GODDINGER, Dieter, 25336 Klein Nordende (DE); HENTRICH, Dirk, 22457 Hamburg (DE)
(86) Internationale Anmeldenummer: PCT/EP2004/001263
(87) Internationale Veröffentlichungsnummer: WO 2004/073664

(56) Entgegenhaltungen:
- EP-A1- 1 004 291
- WO-A2-02/11685
- FR-A1- 2 765 479
- US-A- 5 756 436
- COSMETICS & TOILETRIES, 1997, Seite 197,

## Beschreibung

Die Erfindung betrifft kosmetische Haarbehandlungsmittel, insbesondere Haarpflegemittel, auf der Basis einer speziellen Wirkstoffkombination, ein Verfahren zur Verringerung von Spliss sowie die Verwendung der Wirkstoffkombination in Haarbehandlungsmitteln.

Das menschliche Haupthaar wird heutzutage mit einer Vielfalt kosmetischer Zubereitungen behandelt, die das Haar glänzend und gut kämmbar machen sollen. Durch die vielfältige Behandlung des Haares, beispielsweise bei Bleichung, Färbung, Tönung und Verformung, kann es jedoch zu einer unerwünschten Beeinträchtigung der Haarstruktur kommen. Die Beeinträchtigung der Haarstruktur macht sich beispielsweise in einer schlechten Nass- und Trockenkämmbarkeit, einer verstärkten elektrostatischen Aufladung, verstärkter Sprödigkeit, verringerter Höchstreißkraft und Reißdehnung der Haare, Spliss und einem insgesamt verschlechterten äußeren Erscheinungsbild der Haare bemerkbar.

Eine übliche Möglichkeit zur Milderung dieser Misstände bietet beispielsweise die Nachbehandlung der Haare mit Zusammensetzungen auf der Basis kationischer Polymere oder wasserunlöslicher, nicht-flüchtiger Silikonöle.

Kationische Polymere alleine vermögen nicht immer einen zufriedenstellenden Konditioniereffekt zu bewirken, insbesondere in Bezug auf die Weichheit und die Nass- und Trockenkämmbarkeit von Haaren.

Unlösliche, nicht-flüchtige Silikonöle hingegen sind als gute Konditioniermittel in Haarpflegemitteln weit verbreitet, doch hinterlassen sie die Haare oft mit schlechtem, schwerem und fettigem Griff.

Aus diesen Gründen wurden im Stand der Technik zahlreiche Versuche unternommen, diese beiden Komponenten in Haarpflegemitteln miteinander zu kombinieren, um deren positive Effekte zu addieren und deren Nachteile weitgehend auszuräumen. Beispielsweise in der EP 892 631 B1 wurden Konditioniershampoos vorgeschlagen, die eine Kombination spezieller kationischer Polymere, unlöslicher Silikone und spezieller Tenside umfassen und das Haar noch besser zu konditionieren vermögen. Ähnliche Ergebnisse sind auch aus den Dokumenten WO 92/10162 A1, WO 94/031515 A1 und WO 95/22311 A1 bekannt.

In der EP 1 080 714 A1 wurde eine "2in1"-Zusammensetzung vorgeschlagen, die ein wasserlösliches Silikon, mindestens ein kationisches Polymer und mindestens ein Tensid umfasst und insbesondere in Bezug auf die Weichheit und die Nass- und Trockenkämmbarkeit einen besseren Konditioniereffekt aufweist.

Die Anmeldungen FR-A1-2 765 479 und EP-A1-1 004 291 betreffen Haarwasch- und -konditioniermittel, die vorzugsweise ein nicht-flüchtiges, wasserunlösliches Silikon, ein kationisches Polymer und fakultativ weitere Inhaltsstoffe (u.a. Vitamine) enthalten. Die Mittel dieser Anmeldungen verleihen den Haaren mehr Glätte, Weichheit und Volumen.

Eine Rezeptur aus "Cosmetics & Toiletries", 1997, Seite 197, offenbart ein Haarkonditioniermittel auf der Basis einer Kombination aus einem nicht-flüchtigen

Es besteht dennoch weiterhin der Bedarf nach Haarbehandlungsmitteln, die Haarschädigungen infolge äußerer Einwirkungen und aggressiver kosmetischer Behandlungen wie Bleichen, Färben oder Verformen verhindern oder verringern. Insbesondere wird die Herstellung von Mitteln angestrebt, die eine Splissbildung signifikant reduzieren oder dieser vorbeugen können. Gleichzeitig soll die Nass-und Trockenkämmbarkeit verbessert werden.

Bei Verwendung von Silikonölen ist besonders wichtig, dass kein build-up-Effekt, also keine dauerhafte Belastung der Haare, auftritt und dass das Silikonöl keine nichtflüchtigen Bestandteile enthält.

Überraschenderweise wurde nun eine Wirkstoffkombination gefunden, die diese Erfordernisse in hohem Masse erfüllt.

Gegenstand der Erfindung sind daher kosmetische Haarbehandlungsmittel, die eine Wirkstoffkombination aus
(A) mindestens einem wasserunlöslichen, flüchtigen Silikon,
(B) mindestens einem kationischen Polymer und
(C) mindestens einem weiteren Haarpflegestoff, ausgewählt aus der Gruppe der Vitamine und/oder Provitamine und/oder der kationisch derivatisierten Proteinhydrolysate
enthalten.

Unter wasserunlöslichen Silikonen im Sinne der Erfindung werden solche Silikone verstanden, die bei 20°C zu maximal 0,5% in Wasser löslich sind.

Unter flüchtigen Silikonen, im Sinne der Erfindung sind solche zu verstehen, die vollständig verdampfen und keinen Rückstand auf dem Substrat hinterlassen. Bevorzugt sind solche Silikone, von denen eine Probe von 10 g auf einer Petrischale von 60° C innerhalb von 120 Minuten zu 5-90% verdampft. Besonders bevorzugt sind solche Silikone, die bei diesen Bedingungen zu 10-50% verdampfen.

Erfindungsgemäß bevorzugte Silikonverbindungen sind solche nach Formel (1), 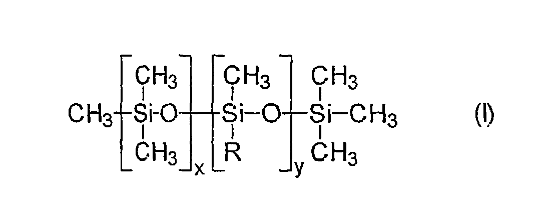 in der x steht für eine Zahl von 1 bis 10, y steht für eine Zahl von 1 bis 10 und R steht für einen Alkylrest mit 2 bis 10 C-Atomen.

Insbesondere bevorzugt sind solche Silikonverbindungen nach Formel (I), in denen die Zahlen x und y für die Zahl 1 stehen. '

Insbesondere bevorzugte Silikonkomponenten im Sinne der Erfindung sind die Verbindungen Hexyl Methicone und Caprylyl Methicone, wie sie beispielsweise unter den Handelnamen SilCare 41M10 und SilCare 41M15 von der Firma Clariant im Handel erhältlich sind.

Die Silikonkomponenten werden in den erfindungsgemäßen Mitteln üblicherweise in einer Menge von 0,001 bis 20 Gew.-%, bevorzugt in einer Menge von 0,1 bis 10 Gew.-% und insbesondere in einer Menge von 0,5 bis 2 Gew.-%, jeweils bezogen auf das Gesamtgewicht des Mittels, eingesetzt.

Bevorzugte kationische Polymere im Sinne der Erfindung sind beispielsweise kationische Cellulosederivate, wie z.B. eine quaternierte Hydroxyethylcellulose, die unter der Bezeichnung Polymer JR^{®} 400 von Amerchol erhältlich ist, kationische Stärke, Copolymere von Diallylammoniumsalzen und Acrylamiden, quaternierte Vinylpyrrolidon/Vinyl-imidazol-Polymere, wie z.B. Luviquat^{®} (BASF), Kondensationsprodukte von Polyglycolen und Aminen, quaternierte Collagenpolypeptide, wie beispielsweise Lauryldimonium hydroxypropyl hydrolyzed collagen (Lamequat^{®}L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine^{®}/Sandoz), Copolymere der Acrylamid mit Dimethyldiallyl-ammoniumchlorid (Merquat^{®} 550/Chemviron), Homopolymere des Dimethyldiallyl-ammoniumchlorids (Merquat^{®} 100), Polyaminopolyamide, wie z.B. beschrieben in der FR-A 2252840 sowie deren vernetzte wasserlöslichen Polymere, kationische Chitinderivate, wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar^{®} CBS, Jaguar^{®} C-17, Jaguar^{®} C-16 der Firma Celanese, quaternierte Ammoniumsalz-Polymere, wie z.B. Mirapol® A-15, Mirapol^{®} AD-1, Mirapol^{®} AZ-1 der Firma Miranol.

Insbesondere bevorzugte kationische Polymere sind kationische Guar-Derivate, kationische Cellulose-Derivate, Homopolymere des Dimethyldiallylammoniumchlorids sowie Copolymere des Dimethyldiallylammoniumchlorids mit Acrylamid.

Die kationischen Polymere werden in den erfindungsgemäßen Zusammensetzungen üblicherweise in einer Menge von 0,001 bis 20 Gew.-%, bevorzugt in einer Menge von 0,01 bis 5 Gew.-% und insbesondere in einer Menge von 0,1 bis 2 Gew.-%, jeweils bezogen auf das Gesamtgewicht des Mittels, eingesetzt.

In einer ersten bevorzugten Ausführungsform der Erfindung wird als Haarpflegekomponente (C) ein Vitamin und/oder Provitamin und/oder ein physiologisch verträgliches Derivat dieser Stoffe eingesetzt.

Dabei sind erfindungsgemäß solche Vitamine, Pro-Vitamine und Vitaminvorstufen bevorzugt, die üblicherweise den Gruppen A, B, C, E, F und H zugeordnet werden.

Zur Gruppe der als **Vitamin A** bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäß verwendeten Zubereitungen enthalten die Vitamin A-Komponenten bevorzugt in Mengen von 0,05-1 Gew.-%, bezogen auf die gesamte Zubereitung.

Zur **Vitamin B**-Gruppe oder zu dem Vitamin B-Komplex gehören u. a.
Vitamin B₁ (Thiamin)
Vitamin B₂ (Riboflavin)
Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendetenen Mitteln bevorzugt in Mengen von 0,05 bis 1 Gew.-%, bezogen auf das gesamte Mittel, enthalten ist.
Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäß verwendeten Mitteln bevorzugt in Mengen von 0,05 - 10 Gew.-%, bezogen auf das gesamte Mittel, enthalten. Mengen von 0,1 - 5 Gew.-% sind besonders bevorzugt.
Vitamin B₆ (Pyridoxin sowie Pyridoxamin und Pyridoxal).

**Vitamin C** (Ascorbinsäure). Vitamin C wird in den erfindungsgemäß verwendeten Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

**Vitamin E** (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäß verwendeten Mitteln bevorzugt in Mengen von 0,05-1 Gew.-%, bezogen auf das gesamte Mittel, enthalten.

**Vitamin F.** Unter dem Begriff "Vitamin F" werden üblicherweise essentielle Fettsäuren, insbesondere Linolsäure, Linolensäure und Arachidonsäure, verstanden.

**Vitamin H.** Als Vitamin H wird die Verbindung (3a*S*,4*S*,6a*R*)-2-Oxohexahydrothienol[3,4-*d*]-imidazol-4-valeriansäure bezeichnet, für die sich aber inzwischen der Triviainame Biotin durchgesetzt hat. Biotin ist in den erfindungsgemäß verwendeten Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäß verwendeten Zubereitungen Vitamine, Provitamine und Vitaminvorstufen aus den Gruppen A, B, E und H. Selbstverständlich können auch mehrere Vitamine und Vitaminvorstufen gleichzeitig enthalten sein.

Pantolacton, Pyridoxin und seine Derivate sowie Nicotinsäureamid und Biotin, aber insbesondere Panthenol und seine physiologisch verträglichen Derivate sind besonders bevorzugt.

Gemäß einer zweiten bevorzugten Ausführungsform der Erfindung wird als Haarpflegekomponente (C) ein kationisch derivatisiertes Proteinhydrolysat eingesetzt.

Dabei handelt es sich erfindungsgemäß um Proteinhydrolysate vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder von biotechnologisch gewonnenen Proteinhydrolysaten. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsatzen wie beispielsweise N,N-Dimethyl-N-(n-Alkyl)-N-(2-hydroxy-3-chloro-n-propyl)-ammoniumhalogeniden durchgeführt. Darüber hinaus können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17^{th} Street, N.W., Suite 300, Washington, DC 20036-4702) genannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein.

Ganz besonders bevorzugt sind die kationischen Proteinhydrolysate und -derivate aus Keratin, Collagen, Elastin, Soja, Milch, Weizen, Seide und Mandel; insbesondere bevorzugt ist Hydroxypropyl Hydrolized Wheat Protein, wie es beispielsweise unter dem Handelsnamen Gluadin WQ von der Firma Cognis im Handel erhältlich ist.

In den erfindungsgemäßen Mitteln sind die Proteinhydrolysate und deren Derivate in Mengen von 0,01 - 10 Gew.-%, bezogen auf das gesamte Mittel, enthalten.

Mengen von 0,1 bis 5 Gew.%, insbesondere 0,1 bis 3 Gew.-%, sind ganz besonders bevorzugt.

In einer dritten bevorzugten Ausführungsform der Erfindung wird als Komponente (C) ein Gemisch aus Vitaminen oder Provitaminen und kationisch derivatisierten Proteinhydrolysaten eingesetzt.

Eine besonders bevorzugtes Komponentengemisch (C) ist erfindungsgemäß die Mischung aus Panthenol oder einem physiologisch verträglichen Derivat des Panthenols mit Hydroxypropyl Hydrolized Wheat Protein.

Erfindungsgemäß beträgt der Gesamtgehalt der Komponente (C) in den erfindungsgemäßen Mitteln 0,01 bis 10 Gew.-%, insbesondere 0,1 bis 5 Gew.-%, jeweils bezogen auf das Gesamtgewicht des Mittels.

Die erfindungsgemäße Wirkstoffkombination kann erfindungsgemäß sowohl in Haarbehandlungsmitteln eingesetzt werden, die nach der Anwendung aus dem Haar wieder ausgespült werden, als auch in solchen Haarbehandlungsmitteln, die auf dem Haar verbleiben.

Zubereitungen, die üblicherweise nach der Anwendung aus dem Haar wieder ausgespült werden sind beispielsweise Shampoos, Spülungen und Konditioniersowie Well- und Färbemittel. Produkte, die üblicherweise im Haar verbleiben, sind beispielsweise Haarfestiger, Haarkuren und Fönwellpräparate. Je nach Art der Zubereitung können neben den erfindungsgemäß obligatorischen Komponenten alle für den jeweiligen Anwendungszweck geeigneten, üblichen Hilfs- und Zusatzmittel eingesetzt werden.

In einer bevorzugten Ausführungsform der Erfindung wird die Wirkstoffkombination als Shampoo konfektioniert.

Shampoos enthalten neben der Basis Wasser üblicherweise anionische, nichtionische kationische, amphotere und/oder zwitterionische Tenside, Perlglanzmittel, Parfümkomponenten, Mittel zur Einstellung des pH-Wertes, Farbstoffe, Konservierungsmittel, gegebenenfalls Ölkörper sowie Komponenten zur Einstellung der Viskosität.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)_{X}-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-(OCH₂-CH₂)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (II), 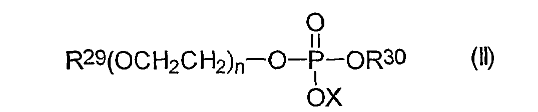 in der R²⁹ bevorzugt für einen aliphatischen Kohlenwasserstoffrest mit 8 bis 30 Kohlenstoffatomen, R³⁰ für Wasserstoff, einen Rest (CH₂CH₂O)ₙR²⁹ oder X, n für Zahlen von 1 bis 10 und X für Wasserstoff, ein Alkali- oder Erdalkalimetall oder NR³¹R³²R³³R³⁴, mit R³¹ bis R³⁴ unabhängig voneinander stehend für einen C₁ bis C₄ - Kohlenwasserstoffrest, steht,
- sulfatierte Fettsäurealkylenglykolester der Formel (III),

   R³⁵CO(AlkO)ₙSO₃M (III)

   in der R³⁵CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind, Monoglyceridsulfate und Monoglyceridethersulfate der Formel (IV), wie sie beispielsweise in der EP-B1 0 561 825, der EP-B1 0 561 999, der DE-A1 42 04 700 oder von A.K.Biswas et al. in J.Am.Oil.Chem.Soc. 37, 171 (1960) und F.U.Ahmed in J.Am.Oil.Chem.Soc. 67, 8 (1990) beschrieben worden sind 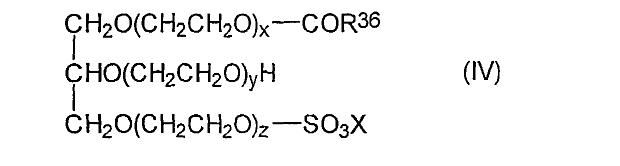 und in der R³⁶CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vor-zugsweise werden Monoglyceridsulfate der Formel (IV) eingesetzt, in der R³⁶CO für einen linearen Acylrest mit 8 bis 18 Kohlenstoffatomen steht

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül und Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniurnglycinat, N-Acylaminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - A-cylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (V)

   R³⁷CO-(OCH₂CH₂R³⁸)_{W}OR³⁹ (V)

   in der R³⁷CO für einen linearen oder verzweigten, gesättigten und/oder ungesättigten Acylrest mit 6 bis 22 Kohlenstoffatomen, R³⁸ für Wasserstoff oder Methyl, R³⁹ für lineare oder verzweigte Alkylreste mit 1 bis 4 Kohlenstoffatomen und w für Zahlen von 1 bis 20 steht,
- Aminoxide,
- Hydroxymischether, wie sie beipielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide,
- Alkylpolygykoside entsprechend der allgemeinen Formel RO-(Z)ₓ wobei R für Alkyl, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.
   Besonders bevorzugt sind solche Alkylpolyglykoside, bei denen R
   - im wesentlichen aus C₈- und C₁₀-Alkylgruppen,
   - im wesentlichen aus C₁₂- und C₁₄-Alkylgruppen,
   - im wesentlichen aus C₈- bis C₁₆-Alkylgruppen oder
   - im wesentlichen aus C₁₂- bis C₁₆-Alkylgruppen oder
   - im wesentlichen aus C₁₆ bis C₁₈-Alkylgruppen besteht.

   Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.
   Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt.
   Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die Tenside werden in Mengen von 0,1 - 45 Gew.%, bevorzugt 1 - 30 Gew.% und ganz besonders bevorzugt von 1 - 15 Gew.%, bezogen auf das gesamte Mittel, eingesetzt.

In einer bevorzugten Ausführungsform können nicht-ionische, zwitterionische und/oder amphotere Tenside sowie deren Mischungen bevorzugt sein.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer weiteren bevorzugten Ausführungsform kann die Wirkung des erfindungsgemäßen Wirkstoffes durch Emulgatoren gesteigert werden. Solche Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensarnen (z.B. Sojabohnen) gewonnen werden, verstanden.
- Fettsäureester von Zuckern und Zuckeralkoholen, wie Sorbit
- Polyglycerine und Polyglycerinderivate wie beispielsweise Polyglycerinpoly-12-hydroxystearat (Handelsprodukt Dehymuls^{®} PGPH)
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

Als Perlglanzwachse kommen beispielsweise in Frage: Alkylenglycolester; Fettsäurealkanolamide; Partialglyceride; Ester von mehrwertigen, gegebenenfalls hydroxysubstituierte Carbonsäuren mit Fett-alkoholen mit 6 bis 22 Kohlenstoffatomen; Fettstoffe, wie beispielsweise Fettalkohole, Fettketone, Fettaldehyde, Fettether und Fettcarbonate, die in Summe mindestens 24 Kohlenstoffatome aufweisen; Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimer-diol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv® TN), Dialkylether, Ringöffnungsprodukte von epoxidierten Fettsäureestern mit Polyolen, Siliconöle und/oder aliphatische bzw. naphthenische Kohlenwasserstoffe in Betracht.

In einer weiteren bevorzugten Ausführungsform der Erfindung wird die Wirkstoffkombination als Haarspülung konfektioniert.

Haarspülungen enthalten neben der Basis Wasser quartäre Ammonioumverbindungen (wie sie in den vorangegangenen Abschnitten bereits beschrieben worden sind), Fettalkohole, Konsistenzgeber, Emulgatoren, Lösungsvermittler, Mittel zur Einstellung der Viskosität und Parfümöle.

In einer weiteren bevorzugten Ausführungsform der Erfindung wird die Wirkstoffkombination als Haarfestiger konfektioniert.

Haarfestiger enthalten neben Wasser niedere Alkohole, Tenside, bevorzugt nichtionische Tenside (wie sie in den vorangegangenen Abschnitten bereits beschrieben worden sind), Stabilisatoren, kationische, nichtionische und/oder anionische Polymere, Mittel zur Einstellung des pH-Wertes sowie, falls als Schaumfestiger formuliert, Treibmittel.

Bei den anionischen Polymeren, welche die Wirkung der erfindungsgemäßen Wirkstoffkombination unterstützen können, handelt es sich um ein anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik^{®}11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze^{®} QM im Handel erhältlich.

Weiterhin können als Polymere zur Steigerung der Wirkung des erfindungsgemäßen Wirkstoffes amphotere Polymere als Bestandteil eingesetzt. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO⁻- oder -SO₃⁻-Gruppen enthalten, und solche Polymere zusammengefaßt, die -COOH- oder SO₃H-Gruppen und quartäre Ammoniumgruppen enthalten.

Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Bevorzugt eingesetzte amphotere Polymere sind solche Polymerisate, die sich im wesentlichen zusammensetzen aus
(a) Monomeren mit quartären Ammoniumgruppen der allgemeinen Formel (VI),

   R²²-CH=CR²³-CO-Z-(CₙH₂ₙ)-N⁽⁺⁾R²⁴R²⁵R²⁶ A⁽⁻⁾ (VI)

   in der R²² und R²³ unabhängig voneinander stehen für Wasserstoff oder eine Methylgruppe und R²⁴, R²⁵ und R²⁶ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist
   und
(b) monomeren Carbonsäuren der allgemeinen Formel (VII),

   R²⁷-CH=CR²⁸-COOH (VII)

   in denen R²⁷ und R²⁸ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen R²⁴, R²⁵ und R²⁶ Methylgruppen sind, Z eine NH-Gruppe und A⁽⁻⁾ ein Halogenid-, Methoxysulfat- oder Ethoxysulfat-Ion ist; Acrylamidopropyl-trimethylammoniumchlorid ist ein besonders bevorzugtes Monomeres (a). Als Monomeres (b) für die genannten Polymerisate wird bevorzugt Acrylsäure verwendet.

Die erfindungsgemäßen Mittel können in einer dritten Variante weiterhin nichtionogene Polymere enthalten.

Geeignete nichtionogenen Polymere sind beispielsweise:
- Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) vertrieben werden.
- Schellack
- Polyvinylpyrrolidone, wie sie beispielsweise unter der Bezeichnung Luviskol^{®} (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.
- Glycosidisch substituierte Silicone gemäß der EP 0612759 B1.

Es ist erfindungsgemäß auch möglich, daß die verwendeten Zubereitungen mehrere, insbesondere zwei verschiedene Polymere gleicher Ladung und/oder jeweils ein ionisches und ein amphoteres und/oder nicht ionisches Polymer enthalten.

Die Polymere sind in den erfindungsgemäß verwendeten Mitteln bevorzugt in Mengen von 0,01 bis 10 Gew.-%, bezogen auf das gesamte Mittel, enthalten. Mengen von 0,1 bis 5, insbesondere von 0,1 bis 3 Gew.-%, sind besonders bevorzugt.

In einer weiteren bevorzugten Ausführungsform der Erfindung wird die Wirkstoffkombination als Haarfärbemittel konfektioniert.

Haarfärbemittel enthalten neben der Basis Wasser direktziehende Farbstoffe und/oder Vorprodukte für Oxidationsfarbstoffe (Kuppler- und EntwicklerKomponenten), Tenside, Fettalkohole und Mittel zur Einstellung des pH-Wertes.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis(2'-hydroxyethyl)-p-phenylendiamin, 2-(2',5'-Diaminophenyl)-ethanol, 2-(2',5'-Diaminophenoxy)-ethanol, 4-Amino-3-methylphenol, 2-Aminomethyl-4-aminophenol, 4-Amino-2-diethylaminomethylphenol, 2-Hydroxy-4,5,6-triaminopyrimidin, 2,4-Dihydroxy-5,6-diaminopyrimidin, 2,5,6-Triamino-4-hydroxypyrimidin und N,N'-Bis-(2'-hydroxyethyl)-N,N'-bis-(4'-aminophenyl)-1,3-diamino-propan-2-ol.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methylpyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, HC Orange 1, Disperse Orange 3, HC Red 1, HC Red 3, HC Red 13, HC Red BN, Basic Red 76, HC Blue 2, HC Blue 12, Disperse Blue 3, Basic Blue 7, Basic Blue 26, Basic Blue 99, HC Violet 1, Disperse Violet 1, Disperse Violat 4, Basic Violet 2, Basic Violet 14, Acid Violet 43, Disperse Black 9, Acid Black 52, Basic Brown 16 und Basic Brown 17 bekannten Verbindungen sowie 1,4-Bis-(β-hydroxyethyl)-amino-2-nitrobenzol, 3-Nitro-4-(β-hydroxyethyl)-aminophenol, 4-Amino-2-nitrodiphenylamin-2'-carbonsäure, 6-Nitro-1,2,3,4-tetrahydrochinoxalin, 2-Hydroxy-1,4-naphthochinon, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Weitere in den erfindungsgemäßen Färbemitteln enthaltene Farbstoffkomponenten können auch Indole und Indoline, sowie deren physiologisch verträgliche Salze, sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

In einer weiteren bevorzugten Ausführungsform der Erfindung wird die Wirkstoffkombination als Wellmittel konfektioniert.

Wellmittel enthalten in der Regel neben dem Reduktionsmittel (Thioglycolsäure, Thiomilchsäure) Komponenten zur Einstellung des pH-Wertes, Tenside, Komplexiermittel, Lösungsvermittler, Fettalkohole und Parfümöle.

Weitere Wirk-, Hilfs- und Zusatzstoffe, die in den bevorzugten Ausführungsformen enthalten sein können, sind beispielsweise
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- haarkonditionierende Verbindungen wie Phospholipide, beispielsweise Sojalecithin, Ei-Lecitin und Kephaline, sowie Silikonöle,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oigosaccharide, wie beispielsweise Glucose, Galactose, Fructose, Fruchtzucker und Lactose,
- konditionierende Wirkstoffe wie Paraffinöle, pflanzliche Öle, z. B. Sonnenblumenöl, Orangenöl, Mandelöl, Weizenkeimöl und Pfirsichkernöl sowie quaternierte Amine wie Methyl-1-alkylamidoethyl-2-alkylimidazolinium-methosulfat,
- Entschäumer wie Silikone,
- Farbstoffe zum Anfärben des Mittels,
- Antischuppenwirkstoffe wie Piroctone Olamine, Zink Omadine und Climbazol,
- Wirkstoffe wie Allantoin und Bisabolol, Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie primäre, sekundäre und tertiäre Phosphate,
- Trübungsmittel wie Latex, Styrol/PVP- und Styrol/Acrylamid-Cocpolymere,
- Perlglanzmittel wie Ethylenglycolmono- und -distearat sowie PEG-3-distearat,
- Pigmente,
- Treibmittel wie Propan-Butan-Gemische, N₂O, Dimethylether, CO₂ und Luft,
- Antioxidatien.

Bezüglich weiterer fakultativer Komponenten sowie die eingesetzten Mengen dieser Komponenten wird ausdrücklich auf die dem Fachmann bekannten einschlägigen Handbücher, z.B. die Monographie von K.H. Schrader, Grundlagen und Rezepturen der Kosmetika, 2. Auflage, Hüthig Buch Verlag Heidelberg, 1989, verwiesen.

Ein zweiter Gegenstand der Erfindung ist ein Verfahren zur Verringerung von Spliss, bei dem die erfindungsgemäße Wirkstoffkombination, enthaltend die Komponenten
(A) mindestens ein wasserunlösliches, flüchtiges Silicon der Formel (I), 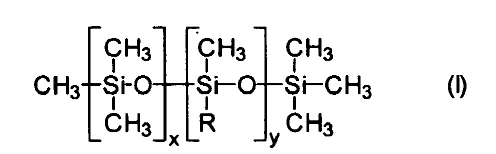 in der x steht für eine Zahl von 1 bis 10, y steht für eine Zahl von 1 bis 10 und R steht für einen Alkylrest mit 2 bis 10 C-Atomen,
(B) mindestens ein kationisches Polymer und
(C) mindestens einen weiteren Haut- und Haar-Pflegestoff, ausgewählt aus der Gruppe der Vitamine und/oder Provitamine und/oder der kationisch derivatisierten Proteinhydrolysate,
auf die trockenen oder feuchten Haare aufgebracht und nach einer Einwirkungszeit gegebenenfalls wieder ausgespült wird.

Ein dritter Gegenstand der Erfindung ist die Verwendung der erfindungsgemäßen Wirkstoffkombination, enthaltend
(A) mindestens ein wasserunlösliches, flüchtiges Silicon der Formel (I), 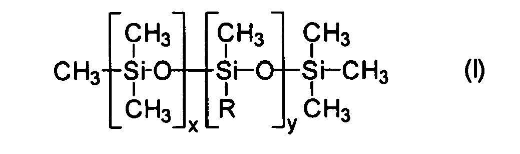 in der x steht für eine Zahl von 1 bis 10, y steht für eine Zahl von 1 bis 10 und R steht für einen Alkylrest mit 2 bis 10 C-Atomen,
(D) mindestens ein kationisches Polymer und
(E) mindestens einen weiteren Haut- und Haar-Pflegestoff, ausgewählt aus der Gruppe der Vitamine und/oder Provitamine und/oder der kationisch derivatisierten Proteinhydrolysate,
in Haarpflegemitteln zur Verringerung von Spliss.

Die folgenden Beispiele sollen den Gegenstand der Erfindung näher erläutern, ohne ihn darauf zu beschränken - alle Gewichtsangaben beziehen sich, sofern nicht anders angegeben, auf Gew.-%.

### 1.) Shampoos

| | A | B | C |
|---|---|---|---|
| | Gew.% | Gew.% | Gew.% |
| Sodium Laureth Sulfate | 10 | 12 | 9 |
| Cocamidopropyl Betaine | 4 | 2 | 6 |
| Disodium Cocoyl Glutamate | 1 | 1 | 2 |
| Cocamidopropyl Aminoxide | 1 | | 2 |
| Coco-Glucoside | | 2 | |
| Behentrimonium Chloride | 1 | 1,5 | 1 |
| Cetyl Alcohol | 0,3 | | 0,3 |
| Stearyl Alcohol | | 0,7 | 0,3 |
| Polyquaternium-10 | 0,1 | 0,2 | 0,1 |
| Glycol Distearate | | | 1 |
| Caprylyl Methicone | 0,5 | 0,6 | 0,4 |
| Laurdimonium Hydroxypropyl Hydrolized Wheat Protein | | 0,5 | |
| Panthenol | 0,5 | | 0,3 |
| Konservierungsmittel | q.s. | q.s. | q.s. |
| Parfüm | q.s. | q.s. | q.s. |
| Wasser | ad 100 | ad 100 | ad 100 |

Das erfindungsgemäße Shampoo zeigte in Haarstudiountersuchungen - d.h. Anwendung des Produktes im sogenannten Halbseitentest unter Verwendung von 2 g Produkt für die Vorwäsche und 3 g Produkt für die Hauptwäsche und anschließender friseurtechnischer Beurteilung - signifikante Vorteile in den Parametern Naßkämmbarkeit, Trockenkämmbarkeit im Vergleich zu marktüblichen Produkten von Wettbewerbern.

Weiterhin wurde die gute Nasskämmbarkeit des erfindungsgemäßen Shampoos im Vergleich zu marktüblichen 2in1-Shampoos über biophysikalische Messungen gezeigt (Meßprinzip: Messung der erforderlichen Kraft zum Durchkämmen von Haarsträhnen. Strähnen Alkinco 6634 mediumblondiert, Messung von 20 Strähnen je Probe und Mittelwertbildung).

| Produkt | eduktion Nasskämmarbeit in % |
|---|---|
| Erfindungsgemässes Shampoo | 56,6 |
| Wettbewerber P (2in1 Shampoo) | 44,5 |
| Wettbewerber GF (2in1 Shampoo) | 32,0 |
| Wettbewerber WG (2in1 Shampoo) | 42,9 |
| Wettbewerber HE (2in1 Shampoo) | 20,7 |

Die Anti-Spliss Wirkung wurde mit folgendem Verfahren ermittelt:
Vorbehandlung der Haarsträhnen :
   Die Haarsträhnen wurden mit einer 10%igen alkalischen Sodium Laureth Sulfate - Lösung 15 Minuten in einem Ultraschallbad vorgereinigt
Produktapplikation :
   Je Produkt wurden 9 Haarsträhnen behandelt, zuzüglich einer Kontrollmessreihe. Hierzu wurden die Haarsträhnen mit Leitungswasser angefeuchtet und jeweils 1 g Produkt pro g Haarsträhne eingearbeitet. Nach einer 5-minütigen Einwirkzeit wurde das Haar 90 Sekunden unter kaltem, fließendern Leitungswasser abgespült. Die Vorkonditionierung der Haare erfolgte bei 25°C und einer relativen Luftfeuchtigkeit von 40% über mindestens 12 Stunden.

### Versuchsmethode:

Die Haarsträhnen wurden in einer klimatisierten Kammer (25°C, 40% relative Luftfeuchtigkeit) 20000 mal gekämmt. Mittels eines Feinstsiebes (Maschenweite 200 µm) wurden hiernach die geschädigten von den ungeschädigten Haaren getrennt und jeweils ausgewogen.

Ergebnis im Vergleich zu marktüblichen 2in1 Shampoos:

| Produkt | pliss-Reduktion bez. auf Nullwert in % |
|---|---|
| Erfindungsgemässes Shampoo | 88,0 |
| Wettbewerber GF (2in1 Shampoo) | 49,7 |
| Wettbewerber LO (2in1 Shampoo) | 55,6 |
| Wettbewerber JLD (3in1 Shampoo) | 21,8 |

### 2.) Spülungen

| | A | B | C |
|---|---|---|---|
| | Gew.% | Gew.% | Gew.% |
| Behentrimonium Chloride | 3,75 | 3,5 | 4,2 |
| Cetylalkohol | 2 | 3 | 1 |
| Stearylalkohol | 1 | | 2 |
| Caprylyl Methicone | 0,8 | 1,0 | 1,2 |
| Polyquaternium-10 | 0,2 | 0,1 | 0,2 |
| Panthenol | 0,5 | | |
| Laurdimonium Hydroxypropyl Hydrolized Wheat Protein | | 1 | 0,5 |
| Konservierungsmittel | q.s. | q.s. | q.s. |
| Parfüm | q.s. | q.s. | q.s. |
| Wasser | ad 100 | ad 100 | ad 100 |

## Patentansprüche

1. Kosmetisches Mittel zur Behandlung, insbesondere zur Pflege, menschlicher Haare, enthaltend eine Wirkstoffkombination aus
(A) mindestens einem wasserunlöslichen, flüchtigen Silicon der Formel (I), 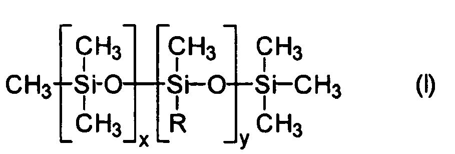 in der x steht für eine Zahl von 1 bis 10, y steht für eine Zahl von 1 bis 10 und R steht für einen Alkylrest mit 2 bis 10 C-Atomen,
(B) mindestens einem kationischen Polymer und
(C) mindestens einem weiteren Haar-Pflegestoff, ausgewählt aus der Gruppe der Vitamine und/oder Provitamine und/oder der kationisch derivatisierten Proteinhydrolysate.

2. Kosmetisches Mittel nach Anspruch 1, **dadurch gekennzeichnet, dass** die Zahlen x und y für die Zahl 1 stehen.

3. Kosmetisches Mittel nach einem der Ansprüche 1 oder 2, **dadurch gekennzeichnet, dass** die Komponente (A) Caprylyl Methicone ist.

4. Kosmetisches Mittel nach einem der Ansprüche 1 bis 3, **dadurch gekennzeichnet, dass** die Komponente (A) in einer Menge von 0,001 bis 20 Gew.-%, bezogen auf das Gesamtgewicht des Mittels, eingesetzt wird.

5. Kosmetisches Mittel nach einem der Ansprüche 1 bis 4, **dadurch gekennzeichnet, dass** das kationische Polymer (B) ausgewählt ist aus den kationischen Guar-Derivaten, den kationischen Cellulose-Derivaten, den Homopolymeren des Dimethyldiallylammoniumchlorid oder den Copolymeren des Dimethyldiallylammoniumchlorids mit Acrylamid.

6. Kosmetisches Mittel nach einem der Ansprüche 1 bis 5, **dadurch gekennzeichnet, dass** das kationische Polymer in einer Menge von 0,001 bis 20 Gew.-%, bezogen auf das Gesamtgewicht des Mittels, eingesetzt wird.

7. Kosmetisches Mittel nach einem der Ansprüche 1 bis 6, **dadurch gekennzeichnet, dass** die Komponente (C) Panthenol oder eines seiner physiologisch verträglichen Derivate ist.

8. Kosmetisches Mittel nach einem der Ansprüche 1 bis 6, **dadurch gekennzeichnet, dass** die Komponente (C) ausgewählt ist aus den kationisch derivatisierten Keratinhydrolysaten, Collagenhydrolysaten, Elastinhydrolysaten, Sojaproteinhydrolysaten, Milchproteinhydrolysaten, Weizenproteinhydroly-saten, Seidenproteinhydrolysaten und Mandelproteinhydrolysaten.

9. Kosmetisches Mittel nach Anspruch 8, **dadurch gekennzeichnet, dass** die Komponente (C) Hydroxypropyl Hydrolized Wheat Protein ist.

10. Kosmetisches Mittel nach einem der Ansprüche 1 bis 6, **dadurch gekennzeichnet, dass** als Komponente (C) ein Gemisch aus Vitaminen oder Provitaminen und kationisch derivatisierten Proteinhydrolysaten eingesetzt wird.

11. Kosmetisches Mittel nach einem der Ansprüche 1 bis 10, **dadurch gekennzeichnet, dass** der Anteil der Komponente (C) in dem gesamten Mittel 0,01 bis 10 Gew.-% beträgt.

12. Verfahren zur Verringerung von Spliss, **dadurch gekennzeichnet, dass** ein kosmetisches Mittel, enthaltend die Wirkstoffkombination aus
(A) mindestens einem wasserunlöslichen, flüchtigen Silicon, der Formel (I), 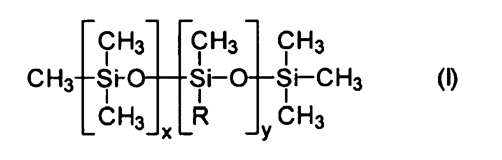 in der x steht für eine Zahl von 1 bis 10, y steht für eine Zahl von 1 bis 10 und R steht für einen Alkylrest mit 2 bis 10 C-Atomen,
(B) mindestens einem kationischen Polymer und
(C) mindestens einem weiteren Haut- und Haar-Pflegestoff, ausgewählt aus der Gruppe der Vitamine und/oder Provitamine und/oder der kationisch derivatisierten Proteinhydrolysate,
auf die trockenen oder feuchten Haare aufgebracht und nach einer Einwirkungszeit gegebenenfalls wieder ausgespült wird.

13. Verwendung einer Wirkstoffkombination aus
(A) mindestens einem wasserunlöslichen, flüchtigen Silicon, der Formel (I), 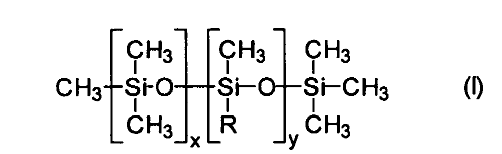 in der x steht für eine Zahl von 1 bis 10, y steht für eine Zahl von 1 bis 10 und R steht für einen Alkylrest mit 2 bis 10 C-Atomen,
(B) mindestens einem kationischen Polymer und
(C) mindestens einem weiteren Haut- und Haar-Pflegestoff, ausgewählt aus der Gruppe der Vitamine und/oder Provitamine und/oder der kationisch derivatisierten Proteinhydrolysate,
in Haarpflegemitteln zur Verringerung von Spliss.

## Claims

1. A cosmetic agent for treating, in particular taking care, of human hair, containing a combination of active ingredients consisting of
(A) at least one volatile water-insoluble silicone of formula (I) 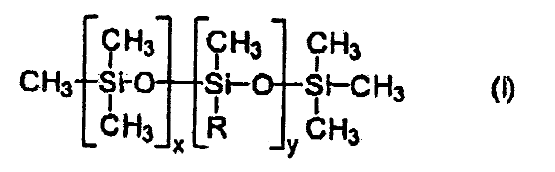 wherein x represents a number from 1 to 10, y represents a number from 1 to 10 and R represents an alkyl radical with 2 to 10 C atoms,
(B) at least one cationic polymer and
(C) at least one further hair care material, selected from the group of vitamins and/or provitamins and/or cationically derivatized protein hydrolysates.

2. The cosmetic agent according to claim 1, **characterized in that** the numbers x and y represent the number 1.

3. The cosmetic agent according to any of claims 1 or 2, **characterized in that** the component (A) is caprylyl methicone.

4. The cosmetic agent according to any of claims 1 to 3, **characterized in that** the component (A) is applied in an amount from 0.001 to 20% by weight, based on the total weight of the agent.

5. The cosmetic agent according to any of claims 1 to 4, **characterized in that** the cationic polymer (B) is selected from cationic guar derivatives, cationic cellulose derivatives, homopolymers of dimethyldiallylammonium chloride or copolymers of dimethyldiallylammonium chloride with acrylamide.

6. The cosmetic agent according to any of claims 1 to 5, **characterized in that** the cationic polymer is applied in an amount from 0.001 to 20% by weight, based on the total weight of the agent.

7. The cosmetic agent according to any of claims 1 to 6, **characterized in that** the component (C) is panthenol or one of its physiologically acceptable derivatives.

8. The cosmetic agent according to any of claims 1 to 6, **characterized in that** the component (C) is selected from cationically derivatized keratin hydrolysates, collagen hydrolysates, elastin hydrolysates, soya protein hydrolysates, lactic protein hydrolysates, wheat protein hydrolysates, silk protein hydrolysates, and almond protein hydrolysates.

9. The cosmetic agent according to claim 8, **characterized in that** the component (C) is hydroxypropyl hydrolyzed wheat protein.

10. The cosmetic agent according to any of claims 1 to 6, **characterized in that** a mixture of vitamins or provitamins and cationically derivatized protein hydrolysates is applied as component (C).

11. The cosmetic agent according to any of claims 1 to 10, **characterized in that** the contents of the component (C) in the whole agent is 0.01 to 10% by weight.

12. A method for reducing split ends, **characterized in that** a cosmetic agent, containing the combination of active ingredients consisting of
(A) at least one water-insoluble, volatile silicon, of formula (I), 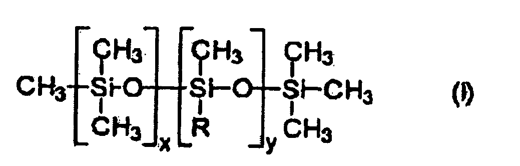 wherein x represents a number from 1 to 10, y represents a number from 1 to 10 and R represents an alkyl radical with 2 to 10 C atoms,
(B) at least one cationic polymer and
(C) at least one further skin and hair care substance, selected from the group of vitamins and/or provitamins and/or cationically derivatized protein hydrolysates,
is applied on dry or wet hair and after a period of action is rinsed again if needed.

13. The use of a combination of active ingredients consisting of
(A) at least one water-insoluble, volatile silicon, of formula (I), 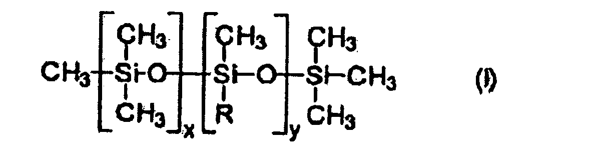 wherein x represents a number from 1 to 10, y represents a number from 1 to 10 and R represents an alkyl radical with 2 to 10 C atoms,
(B) at least one cationic polymer and
(C) at least one further skin and hair care substance, selected from the group of vitamins and/or provitamins and/or of cationically derivatized protein hydrolysates,
in hair care agents for reducing split ends.

## Revendications

1. Agent cosmétique pour traiter, en particulier soigner les cheveux humains, contenant une combinaison de substances actives constituée de
(A) au moins une silicone volatile insoluble dans l'eau de formule (1) 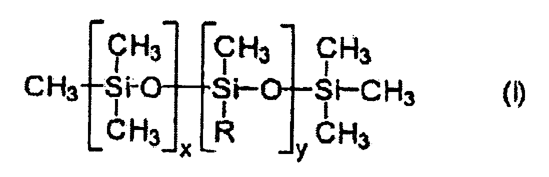 dans laquelle x représente un nombre de 1 à 10, y représente un nombre de 1 à 10 et R représente un radical alkyle ayant de 2 à 10 atomes de C,
(B) au moins un polymère cationique
(C) au moins un agent de soin capillaire supplémentaire, choisi dans le groupe formé par les vitamines et/ou les provitamines, et/ou les hydrolysats de protéine à dérivatisation cationique.

2. Agent cosmétique selon la revendication 1, **caractérisé en ce que** les nombres x et y représentent le nombre 1.

3. Agent cosmétique selon l'une quelconque des revendications 1 ou 2, **caractérisé en ce que**, le composant (A) est la caprylyl-méthicone.

4. Agent cosmétique selon l'une quelconque des revendications 1 à 3, **caractérisé en ce que** le composant (A) est mis en oeuvre en une quantité de 0,001 à 20% en poids, par rapport au poids total de l'agent.

5. Agent cosmétique selon l'une quelconque des revendications 1 à 4, **caractérisé en ce que** le polymère cationique (B) est choisi parmi les dérivés cationiques de guar, les dérivés cationiques de cellulose, les homopolymères du chlorure de diméthyldiallylammonium, ou les copolymères du chlorure de diméthylammonium avec l'acrylamide.

6. Agent cosmétique selon l'une quelconque des revendications 1 à 5, **caractérisé en ce que** le polymère cationique est mis en oeuvre en une quantité de 0,001 à 20% en poids, par rapport au poids total de l'agent.

7. Agent cosmétique selon l'une quelconque des revendications 1 à 6, **caractérisé en ce que** le composant (C) est le panthénol ou l'un de ses dérivés, acceptables sur le plan physiologique.

8. Agent cosmétique selon l'une quelconque des revendications 1 à 6, **caractérisé en ce que** le composant (C) est choisi parmi les hydrolysats de kératine, hydrolysats de collagène, hydrolysats d'élastine, hydrolysats de protéines du soja, hydrolysats de protéines lactiques, hydrolysats de protéines du blé, hydrolysats de protéines de la soie et hydrolysats de protéines des amandes, à dérivatisation cationique.

9. Agent cosmétique selon la revendication 8, **caractérisé en ce que**, le composant (C) est une protéine du blé hydrolysée à groupe hydroxypropyle.

10. Agent cosmétique selon l'une quelconque des revendications 1 à 6, **caractérisé en ce qu'**un mélange de vitamines ou de provitamines et d'hydrolysats de protéine à dérivatisation cationique est mis en oeuvre en tant que composant (C).

11. Agent cosmétique selon l'une quelconque des revendications 1 à 10, **caractérisé en ce que** la teneur du composant (C) dans l'agent total s'élève de 0,01 à 10% en poids.

12. Procédé pour réduire les fourches, **caractérisé en ce qu'**un agent cosmétique, contenant la combinaison de substances actives constituée de
(A) au moins une silicone volatile insoluble dans l'eau de formule (I) 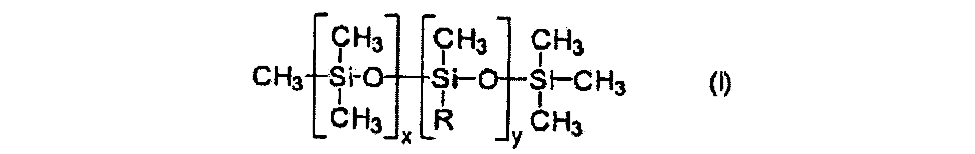 dans laquelle x représente un nombre de 1 à 10, y représente un nombre de 1 à 10 et R représente un radical alkyle ayant de 2 à 10 atomes de C,
(B) au moins un polymère cationique et
(C) au moins un agent de soin cutané et capillaire, supplémentaire, choisi dans le groupe formé par les vitamines et/ou les provitamines et/ou les hydrolysats de protéines à dérivatisation cationique,
est appliqué sur les cheveux secs ou humides et après une durée d'action, est rincé de nouveau si nécessaire.

13. Utilisation d'une combinaison de substances actives constituée de
(A) au moins une silicone volatile, insoluble dans l'eau, de formule (I), 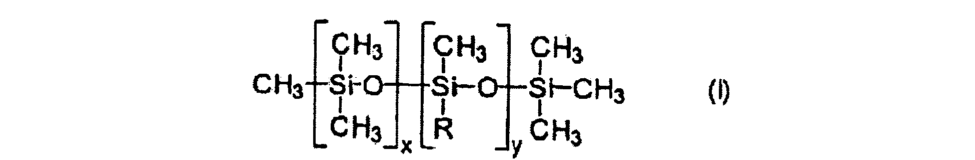 dans laquelle x représente un nombre de 1 à 10, y représente un nombre de 1 à 10 et R représente un radical alkyle ayant de 2 à 10 atomes de C,
(B) au moins un polymère cationique et
(C) au moins un agent de soin cutané et capillaire, supplémentaire, choisi dans le groupe formé par les vitamines et/ou les provitamines, et/ou les hydrolysats de protéines à dérivatisation cationique, dans les agents de soin capillaire pour réduire les fourches.
